# EUROPEAN PATENT SPECIFICATION

(11) **EP 0 388 226 B1**
(45) Date of publication and mention of the grant of the patent: **09.08.1995**
(21) Application number: 90302851.2
(22) Date of filing: 16.03.1990
(51) Int. Cl.: A61K 31/195, A61K 31/23, A61K 31/70, A61K 38/27

(54) **Means for the treatment of senile dementia, memory disorders and related conditions**
Mittel zur Behandlung von seniler Demenz, Gedächtnisstörungen und ähnlichen Zuständen
Moyen de traitement de la démence sénile, des troubles de la mémoire et états comparables

(30) Priority: 16.03.1989 JP 64057/89
(43) Date of publication of application: 19.09.1990
(73) Proprietor: AJINOMOTO CO., INC., Tokyo 104 (JP)
(72) Inventor: Torii, Kunio, c/o Central Research Lab., Kawasaki-ku, Kawasaki-shi, Kanagawa-ken (JP); Oomura, Yutaka, Suita-shi, Osaka-fu (JP); Sasaki, Kazuo, Toyama-shi, Toyama-ken (JP); Kojima, Hiroyuki, c/o Central Research Lab., Kawasaki-ku, Kawasaki-shi, Kanagawa-ken (JP)
(74) Representative: Armitage, Ian Michael

(56) References cited:
- WO-A-85/03869
- US-A- 4 444 760
- RESEARCH COMMUNICATIONS IN PSYCHOLOGY, PSYCHIATRY AND BEHAVIOR, vol. 12, no. 2,1987, pages 105-117; H. LABORIT et al.: "Phorbol'esters antagonize scopolamine - induced amnesia of a passieve avoidance"
- JOURNAL DE PHYSIOLOGIE, vol. 81, no. 4, 1986, pages 252-260; D.L. ALKON et al.:"Biochemical mechanisms of memory storage"
- NEUROBIOLOGY OF AGING, vol. 11, no. 1, January/February 1990, page 77; J.R.CONNOR: "Iron storage and transport proteins in the brain in aging and Alzheimer's disease"
- NATURE, vol. 332, 24th March 1988, pages 360-361; K.J. ANDERSON et al.: "Basic fibroblast growth factor prevents death of lesioned cholinergic neurons invivo"
- THE MERCK INDEX, 11th edition, 1989, page 637, abstract no. 4016, Merck & CO. INC., Rahway, N.J., US
- BRAIN RESEARCH, vol. 452, nos. 1/2, 1988, pages 164-174, Elsevier Science Publishers B.V. (Biomedical Division); G. COLE et al.: "Decreased levels of protein kinase C in Alzheimer brain"
- THE CANADIAN JOURNAL OF NEUROLOGICAL SCIENCES, vol. 15, no. 2, May 1988, page 221; R. GARZA et al.: "Influence des facteurs neuronotrophiques sur ladifférenciation des neurones cholinergiques centraux de rat en culturecellulaire"

## Description

The present invention relates to means for the treatment of senile dementia, memory disorders and related conditions. In particular, the means of the present invention comprises a drug for the treatment of senile dementia, memory disorders and related conditions, which has few adverse side effects. The drug comprises acidic fibroblast growth factor (aFGF), its related analogues, derivatives or functional equivalents thereof. Alternatively or in combination with aFGF, the drug may also comprise substances such as glucose, in combination with one or more L-amino acids (e.g. arginine, leucine, isoleucine or valine) in a form and amount which stimulates the secretion of aFGF in the brain or peripheral tissues. Alternatively or in combination with aFGF the drug may comprise one or more L-amino acids (e.g. arginine, leucine, isoleucine or valine) in a form and amount which stimulates the secretion of aFGF in the brain or peripheral tissues. The use of fibroblast growth factor particuarly acidic fibroblast growth factor in wound healing is disclosed in USP 4,444,760 and The Merck Index, 11th edition, 1989, p637 No 4016. Nature, 332, 24th March, 1988, pp 360-361 described the role of basic fibroblast growth factor in the prevention fo death of lesioned cholinergic neurons *in vivo*. Research Communications in Psychology, Psychiatry and Behaviour, 12, (2), 1987, pp 105-117 describes antagonism of scopolamine-induced amnesia by phorbol esters.

The proportion of elderly people in many countries is getting higher year by year. For example in Japan 10.5% of the total population are older than 65 years of age. At the beginning of the 21st century, approximately 16% of the total population will be older than 65 years of age. As a consequence, the number of people with senile dementia may double, and in Japan the number of patients with the condition could be more than one and a half million. Such a huge number of patients with senile dementia, imposes great burdens on both family and hospital care facilities. Furthermore, the national expenditure for looking after these patients which is already large will inevitably rise considerably. Therefore, in the medical field, the development of an effective therapy for senile dementia is as important an issue as the development of a therapy for cancer and AIDS.

Senile dementia as a consequence of brain ischaemia appears to be the most common form of the condition. It is quite difficult to get complete regression of this form of senile dementia by use of any form of therapeutic care, once the dementia has become established. However, by reducing risk factors, mainly brain haemorrhage and any factors causing a restriction of blood flow, it should be possible to minimise the likelihood of senile dementia. Other factors which render people vulnerable to the condition are e.g. brain hypoxia after carbon monoxide intoxication, heart shock, severe haemorrhage, or the failure of energy metabolism in the brain during hypoglycaemia or prolonged seizure.

Any treatments designed to guard against the development of senile dementia after brain ischaemia and hypoglycaemia, not only need to suppress neuronal death but also to induce neuronal plasticity and aid a reconstruction of the neuronal network by the remaining healthy neurons.

The brain, which is only approximately 2% of body weight, nevertheless needs a blood supply of approximately 15% of the cardiac output and an oxygen supply sufficient to support an oxygen consumption of 20% of the total body consumption.

The energy source in the brain is provided by the aerobic oxidation of glucose supplied from the peripheral blood stream. The brain accounts for approximately 25% of the total glucose expenditure. Despite these heavy demands on oxygen, glucose and energy, the brain's oxygen and glucose storage capacity is small. Therefore, if the supply of these energy sources is stopped, the production of energy for the brain stops for several dozens of seconds with consequent loss of physiological function. If the supply of these energy sources is limited or stopped for a more prolonged period, irreversible brain damage may occur.

Therefore, the brain is very vulnerable to any discontinuities in the supply of energy sources and to any dysfunctions in the metabolism of these energy sources. A similar pattern of brain damage is evoked by for example brain hypoxia, ischaemic hypoglycaemia and continuous seizure.

In such brain damage there is neuron necrosis thought to be induced by the unusual extracellular release of stimulatory neurotransmitters, such as glutamic and aspartic acids. In recent years, several antagonists for these stimulatory neurotransmitters have been developed to protect the brain from damage after spasms etc., but the pharmaceutical efficacy of these antagonists is poor and their effects are generally insufficient to prevent brain dysfunction including dementia.

In gerbils, the major pathological change caused by experimentally induced ischaemia is neuronal death (necrosis). The distribution of neuronal death observed, is quite specific, owing to the particular vulnerability of neurons in the CA1 layer of the hippocampus, the lateral area of the corpus striatum, the 3rd, 5th and 6th layers of the cerebrum cortex and of the Purkinje cells of the cerebellum (Kirino T.: Brain Res., 239, 57. (1982) etc.). The hippocampus, which is composed of the dentate gyrus and, layers of CA3 and CA1, seems to play some important roles both in the acquisition of memory, and the recognition of new information.

When the blood flow to the brain returns to normal after the production of ischaemia by the occlusion of the bilateral carotid artery for a short time, the homeostasis of, and the energy metabolism in, the brain (including the hippocampus) readily recovers (nutritionally and physiologically speaking) concurrent with the normal action potential of the pyramidal cell in the hippocampus (Monaghan, D.H., et. al.: Nature, 306, 176 (1983)). However, the pyramidal cells still become necrotic, from 3 to 4 days after the induction of ischaemia, despite the fact of no lesions being apparent in both the glial cell and the dendritic cells projected into the CA1 layer (Peitio, C.K. et. al.: Neurology, 37, 1281 (1987)).

This phenomenon explains why markedly enhanced extracellular levels of the stimulatory L-amino acid glutamic acid (caused by its higher release and lower uptake after prolonged ischaemia), causes extraordinary neuronal excitation. There is then an influx of extracellular Ca ions followed by an intracellular degradation of lipids and proteins, with final destruction of the neurons being caused by mitochondrial dysfunction and damage. (Benveniste, H., et. al., J. Neurochem, 43, 1369, (1984), Duce, I.R. et. al.: Brain Res., 263, 77 (1983)).

The present invention relates to newly developed drugs for the treatment of senile dementia which have few, if any, adverse side effects. In particular, the drugs render the neurons less susceptible to damage caused by highly enhanced extracellular levels of glutamic acid, with a resultant suppression of neuronal death rate. The drugs also aid the reconstruction of the neuronal network by the induction of plasticity during and after brain ischaemia.

The most vulnerable area of the brain to neuronal death in consequence of brain ischaemia and hypoglycaemia is the hippocampus, and particularly the pyramidal cells in the CA1 layer thereof.

Forebrain ischaemia can be induced in experimental animals by the bilateral occlusion of the carotid artery. When this is done in gerbils for 5 minutes, or in rats for 10-30 minutes, the blood flow readily returns to normal after removal of the occlusion. Both the content of ATP and the energy charge level (as parameters of energy metabolism in the brain) decline rapidly during brain ischaemia and also quickly recover very soon after the cause of ischaemia is removed (e.g. occlusion of carotid arteries). There are no pathological changes in the pyramidal cells of the hippocampal CA1 layer until 24 hours after the induction brain ischaemia.

It is possible to record neuronal electrical activity. In animals in which ischaemia is induced, there is an increase in the number of intracellular impulses per second as compared to normal controls. This data suggests that the pyramidal cells in the hippocampal CA1 layer are still alive at least until the first day subsequent to the induction of ischaemia, but that these neurons lose their electrophysiological responses and fall into a state of functional death around the second day after the induction of ischaemia. Three or four days after the induction of short term brain ischaemia, the pyramidal cells in the hippocampal CA1 layer become necrotic. The development of this brain lesion gradually progress and is called "delayed neuronal death" (as compared to the brain damage caused by a glutamic acid agonist, e.g. kinic acid). Both anaemic and haemorrhagic infarcts in the brain, induced by e.g. brain thrombosis, carbon monoxide intoxication, heart shock, heart failure, severe haemorrhage and so on, are potent stimulators of ischaemic brain damage. Ischaemic brain damage can also be caused by hypoglycaemia, prolonged seizure, (i.e., epilepsy) which adversely affect the energy metabolism in the brain.

In these cases, any drugs which can increase neuronal tolerance to unusually high extracellular levels of glutamic acid or which aid the maintenance of brain homeostasis, would limit the development of brain damage and consequently protect against dementia.

In relation to the discussions above, it has been observed that people who take high protein diets containing large amounts of glutamic acid do not seem to suffer so markedly from the effects of high extracellular concentrations of glutamic acid and consequent histological damage after brain ischaemia.

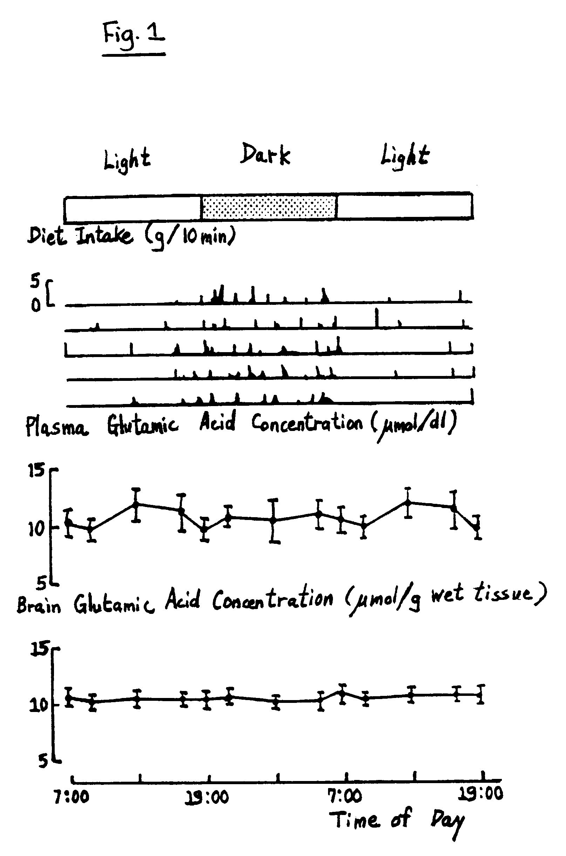

The invention will now be discussed more fully with reference to Figures 1-7 shown by way of example only.

Figure 1 is a typical plot showing the circadian rhythm of glutamic acid in the plasma and brain of male Sprague-Dawley strain rats (N=8), weighing 350g, concurrent with ad-libitum intake of a diet containing 6.8% (w/w) of L-glutamic acid. This data indicates that the levels of glutamic acid in the plasma as well as the brain, are unchanged by the uptake of large amounts of glutamic and aspartic acids during both the digestive and absorptive phases subsequent to ingestion. In addition to this data, it has also been reported that plasma and brain levels of glutamic acid do not increase in mice fed a diet containing 30% (w/w) monosodium glutamate. Furthermore, where high plasma levels of glutamic acid are experimentally produced in dams there appears to be no incremental increase in placental transfer or transfer into milk of glutamic acid. Thus, plasma and brain concentrations of glutamic acid are essentially unchanged by high dietary intakes of glutamate. This is because ingested glutamate is catabolized into alanine and/or glutamine. Furthermore, barrier systems in the alimentary organs, brain, placenta and mammary gland are operative.

In contrast, when infant mice 9 or 10 days of age, received an excessive dose of L-glutamate (i.e., 4g/kg body weight) parenterally or by forced intubation with a concentrated solution, their plasma as well as brain levels of glutamic acid increased to several hundred times more than normal and caused forcal (diffused) necrosis in the vulnerable areas of brain, i.e., the hippocampus, substantial nigra, area postrama, amygdala, olfactory bulb, and hypothalamus (the paraventricular nuclei, the arcuate nuclei, the median eminence). This is because in infants their barrier systems are still immature and unable to sufficiently operate to control brain and plasma levels of glutamic acid in response to high loadings of glutamic acid experimentally induced.

In contrast, brain lesions induced by dietary loading with glutamic acid were markedly less severe when the infant mice were orally pre-treated with certain nutrients, such as milk formula or its ingredients (glucose or L-amino acids), one hour prior to the experimental dietary loading with L-glutamic acid. This was despite the enhancement of plasma glutamic acid levels caused by the treatment. These facts suggest that the cephalic phase of both olfactory and gastric stimuli, subsequent to absorption of nutrient from the alimentary tract along with secretion of humoral factors, such as gastrointestinal hormones and neurotrophic growth factors, enhance the glutamic acid tolerance level in the brain.

Thus, the applicants have discovered that there are changes in neurotrophic factors during as well as after eating. One of these factors, acidic fibroblast growth factor (aFGF) increases to several thousand times normal levels approximately one and a half hours after eating. Another factor is platelet derived growth factor (PDGF) which declines to one-eighth normal levels.

It is known that the sprouting of neurons is elicited by the addition of these growth factors into the culture medium of a neuron culture.

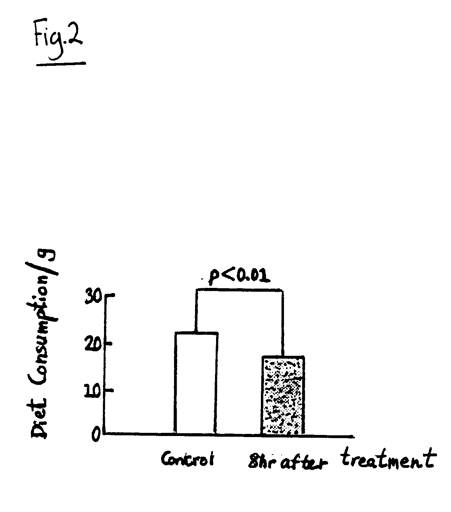

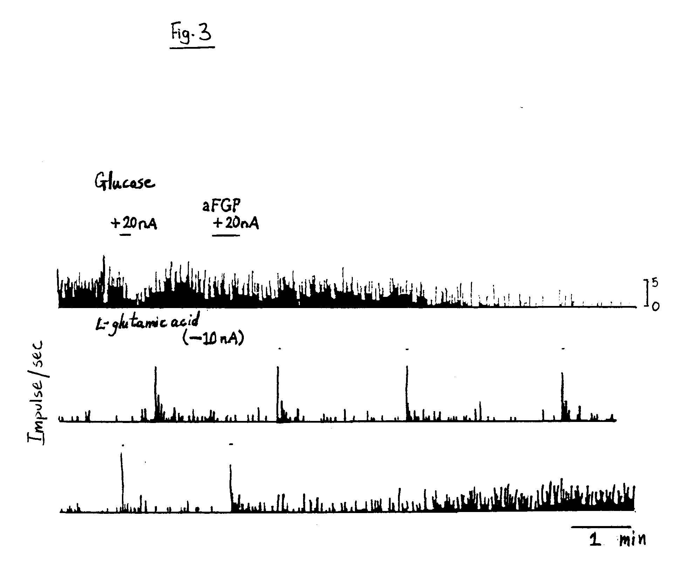

The applicants have examined the effects of administering very low dosages of aFGF and PDGF into the brains of rats. In particular they examined anorexigenic function and the discharge of glucose sensitive neurons. It was found that aFGF alone was potent in decreasing dietary intake and stimulating the spontaneous discharge of glucose sensitive neurons in the lateral hypothalamic area (feeding centre), (Figures 2 and 3). Figure 2 shows that in rats treated with aFGF intra-third ventricularly (220ng/rat), the appetite for food significantly decreased (p<0.01 vs control animal). Figure 3 indicates the chronological spontaneous discharge of glucose sensitive neurons in the feeding centre of the rat i.e. the lateral hypothalamic area. This recoding of neuron discharge employs a very sophisticated method comprising the iontophoretic application of glucose, aFGF and L-glutamic acid to the lateral hypothalamic area of the brain using a multi-barrelled microelectrode. The amounts of materials administered iontophoretically are indicated in Figure 3 on the basis of nano amperes (nA). The pattern of recording suggests that the strong suppression of neural activity occurs after a latency of about eight and a half minutes after aFGF treatment and continues for 15 minutes. The applicants observed a similar phenomenon on the application of some phorbol esters which act as protein-kinase C activators, e.g. 1-oleoyl-2-acetyl-glycerol. In contrast, the suppressive effects stimulated by both the phorbol ester and aFGF are diminished by the application of the protein-kinase C inhibitor, imipramine (10,11-dihydro-N,N-dimethyl-5H-dibenz[b.f]azepine-5-propanamine), suggesting that a physiological function of aFGF is as an activator of protein kinase C.

Histochemical examination, using antibody for the full sequence of aFGF, indicates that when an animal is hungry, aFGF (as indicated by the areas stained by the antibody) is distributed mainly in the ependymal cells. Two hours after eating however, the distribution of aFGF changes and is mainly located in the lateral hypothalamic area, the zona incerta, the hippocampus, the amygdala and the nucleus solitalis. Some ependymal cells lose intracellular aFGF, which appears to move into the brain parenchyma tissue.

The distribution of mRNA for aFGF, in the brain ventricles and the hypothalamus, were examined by the use of in situ hybridisation techniques using cDNA for aFGF. The cDNA comprised a 30 base oligonucleotide probe from the N-terminus which codes for 10 amino acid residues at the N-terminus of the aFGF peptide sequence. mRNA for aFGF was located in the ependymal cells, but not in the neurons of the lateral hypothalamic area. These facts indicate that aFGF, released from the ependymal cells after eating or glucose administration, is diffused in either the cerebrospinal fluid or the brain parenchymal tissue with subsequent incorporation into the brain areas, discussed above. This evidence strongly suggests that aFGF may be a possible neurotrophic factor supporting and maintaining neuron function. In addition, or alternatively, it may induce the plasticity required for the reconstruction of the neural network after brain ischaemia and consequent neuronal death, by stimulating and supporting neuronal growth and development.

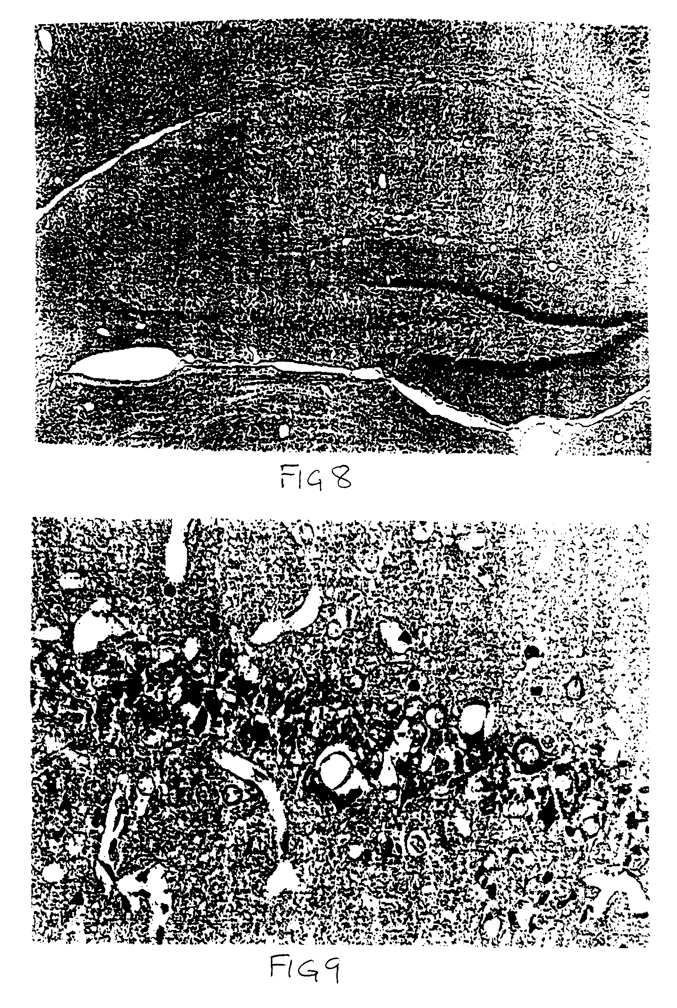

The applicants have examined the survival rate of the pyramidal cells in the hippocampal CA1 layer of two groups of gerbils. In a first experimental group, the gerbils were pre-treated with aFGF, and short term brain ischaemia was experimentally induced. Each gerbil in the experimental group received a dorsal subcutaneous implant of an Alzet's mini-pump containing aFGF in ringer saline (20»g/ml). The pump was cannulated bilaterally into the lateral ventricles to administer aFGF (200ng/animal/day) constantly for 7 days. In a second experimental group, the gerbils received a similar pump implant, short term brain ischaemia was similarly induced, but this group did not receive pre-treatment with aFGF. This second experimental group served as a control group. Three days after the implantation was performed, the carotid arteries of each of the gerbils in both experimental groups were bilaterally occluded for 5 minutes under 2% (v/v) halothane in oxygen gas to induce forebrain ischaemia. Anaesthesia was then immediately terminated and 5 minutes later the occlusion was released to allow recovery of the blood circulation. Each gerbil was fixed and killed by the circulatory perfusion of 2.5% glutaraldehyde in 0.1M phosphate buffer (pH 7.3). The brains were then removed from the carcases. The fixed brains were embedded in paraffin wax, and sagittal sections made. The brain sections (which included the hippocampus), were treated with Nissl's stain, and examined histopathologically. No pathological changes were observed in the hippocampal pyramidal cells of the CA1 layer in the brains from animals in the first experimental group receiving aFGF. However, severe damage was seen in the same cells in the brains of animals in the second experimental group which did not receive aFGF (Figs. 8 and 11).

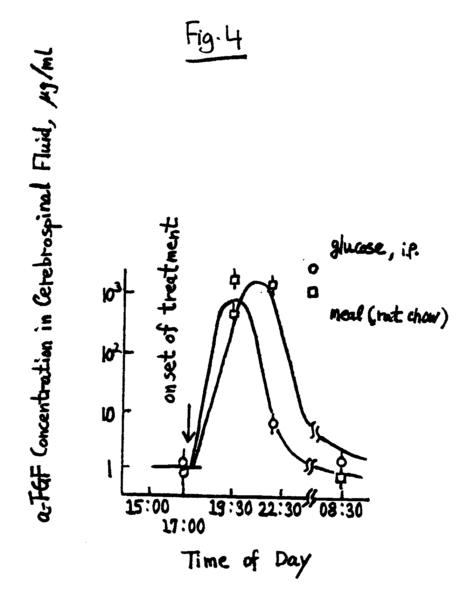

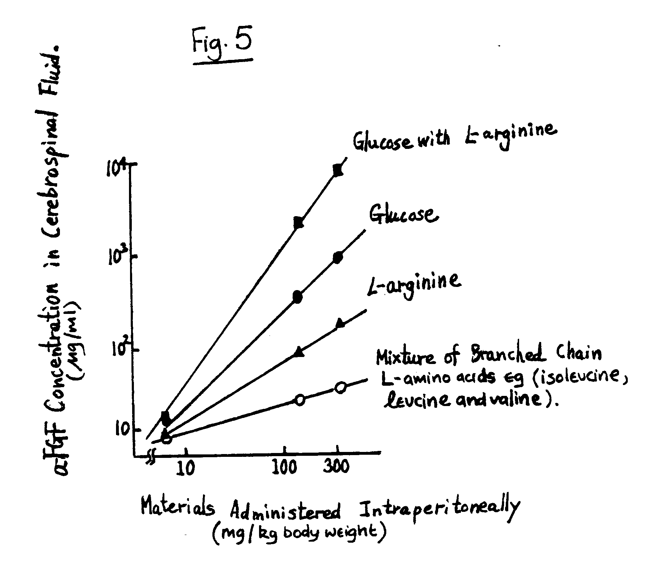

When rats were treated with nutrients such as glucose, L-arginine, L-leucine, L-isoleucine or L-valine either orally or parentally, aFGF release into the cerebrospinal fluid was observed. Cerebrospinal fluid levels of aFGF reached a maximum level approximately 2 hours after treatment (Figs. 4 and 5).

This observation seems to suggest that these nutrients could be administered to a patient recovering from brain ischaemia and/or hypoglycaemia as an alternative to aFGF therapy.

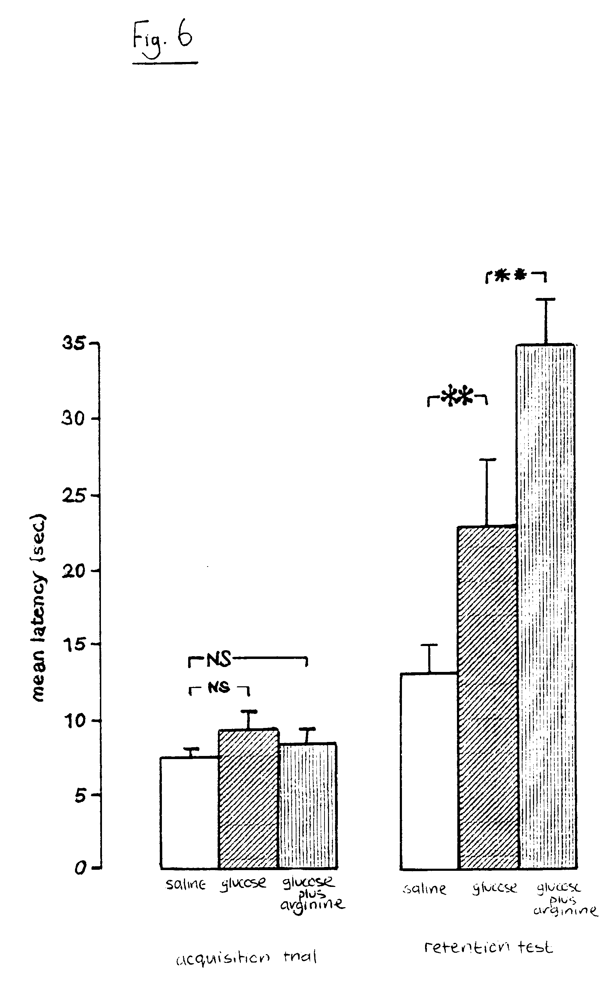

The possible therapeutic effect of these nutrients on patients with a memory function disorder was studied in mice using the passive avoidance test. Each mouse in the test was allowed a period to acclimatise to a specially designed cage, which had separate light and dark compartments. On the second day, each animal received glucose with or without arginine intraperitoneally. Two hours after dosage, each animal was accommodated in the light compartment of the cage. When a mouse moved to enter the dark compartment, it received a weak electric shock as an acquisition trial. On day three, each mouse was similarly accommodated in the light compartment and the time taken to move from the light compartment to the dark compartment was determined. This latency period was measured to give an estimate of memory retention. Data from the passive avoidance test is shown in Figure 6 which shows the effect of pre-training glucose or glucose plus arginine administration on the passive avoidance response in mice. The data indicates that intraperitoneal administrations of glucose with or without arginine, were effective in improving memory retention. The results are similar to those seen in experiments where an L-amino acid e.g. L-leucine, L-isoleucine and L-valine was administered to stimulate the release of aFGF in the brain.

Thus a number of facts have been accumulated by the applicants which together establish the discovery of a new drug useful for the treatment of senile dementia, memory loss and other related conditions. These drugs which appear not to have adverse effects comprise any of: (1) aFGF, (2) compounds related to aFGF such as physiologically active fragments thereof, derivatives thereof and analogue peptides, (3) non-peptide organic chemicals with an active domain physiologically similar to the active domain of aFGF and (4) nutrients e.g., glucose, L-arginine, L-leucine, L-isoleucine and L-valine which when administered in the correct amounts and/or combinations, stimulate the release aFGF. Though treatment with glucose or arginine alone appears to definitely improve the symptoms of patients with brain ischaemia and subsequent dementia, the combination of glucose with an L-amino acid as mentioned above is quite effective for administration to patients immediately after normal blood flow is restored and their energy metabolism has recovered from the effects caused by spasms. When, however, patients are ischaemic or in hypoglycaemic shock continuous administration of aFGF into the brain is desirable.

aFGF can be manufactured by culturing an aFGF-yielding cell or by uses of recombinant DNA techniques well known in the art for the production of polypeptides. Physiologically active fragments of aFGF can be produced from aFGF by the enzymatic degradation or chemical treatment of aFGF. Analogue peptides of aFGF, for example, Ala-aFGF and Met-aFGF, can be prepared using the techniques of recombinant DNA technology.

In addition, there is a large group of non-peptide organic chemicals with active domain physiologically similar to that of aFGF. Such a group comprises the phorbol esters e.g. 1-oleoyl-2-acetyl-glycerol etc. The phorbol esters can be chemically or enzymatically synthesized.

The prescriptive administration of these drugs for patients with senile dementia, memory loss and other related conditions, has to be carefully controlled with respect to e.g. dosage, form of drug, and administration route. The prescriptive administration will depend upon the exact physiological state of a patient particularly with respect to energy metabolism, blood circulation and the time after brain ischaemia and hypoglycaemia. For example, constant infusion of aFGF into the cerebrospinal fluid (1 to 200ug/person/day) should be used where a patient has just suffered from spasms. Two days later, the therapy should be combined with the administration of nutrients parenterally or orally, i.e., glucose and/or L-amino acids (e.g. arginine, leucine, isoleucine and valine), at a dose ranging from 1 to 100g/person/day, if possible, less than 60g/person/day.

aFGF can be prepared as a pharmaceutical wherein it is the sole active ingredient or it can be presented in combination (optionally for sequential administration) with glucose and/or one or more L-amino acids, e.g. those mentioned above. Typically, the drug will be presented as: (1) a pharmaceutical product containing one or more compounds presented as a powder, granules, tablets, sugar-coated tablets, capsules, liquors; or (2) a formulation for e.g. intravenous or alimentary administration. However, aFGF, fragments and derivatives thereof and related analogue peptides, are suitable for administration directly into the brain.

The invention will now be further exemplified with reference to the following examples.

### Example 1

Brain ischaemia was induced in male gerbils, weighing around 250g, by the bilateral occlusion of the carotid arteries for 5 minutes. The occlusion was then released and the blood circulation allowed to normalise. Five days after this treatment, the brain of each animal was fixed by the perfusion of 2.5% (v/v) glutaraldehyde in 0.1M phosphate buffer (pH 7.3). Saggital sections of the brain (including the hippocampus) stained with Nissl's stain were made for pathological examination. Lesions, with the necrosis of pyramidal cells in the hippocampal CA1 layer were observed.

Similar male animals, weighing around 250g, received subcutaneous dorsal implants comprising an Alzet's mini-pump (2002 type). The pump administered aFGF (20ug/ml) in saline containing heparin (50ug/ml) at 0.5»l/hr for 7 days.

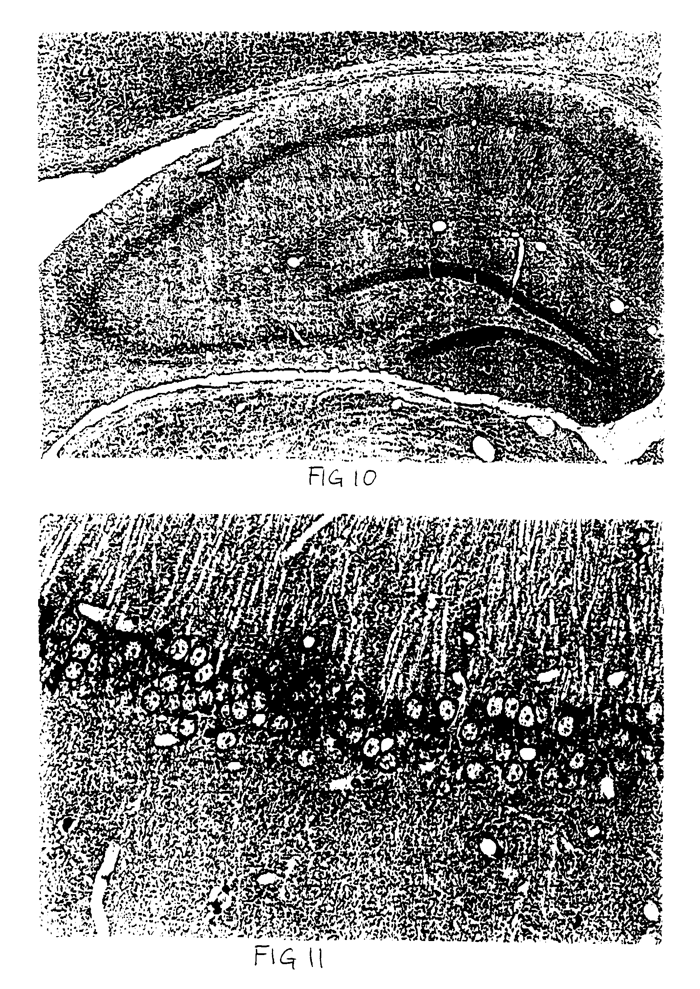

Three days after receipt of the surgical implant, each animal was placed under parnene (halothane) anaesthesia and brain ischaemia was induced by the bilateral occlusion of the carotid arteries for 5 minutes. When forebrain ischaemia was induced, anaesthesia was immediately discontinued. Five days after this treatment, the animals were sacrificed by use of the pentobarbiturate Nembutal (20mg/kg body weight, i.p.), and their brains fixed by the perfusion of glutaraldehyde solution. The fixed brains were embedded in paraffin wax. Serial sagittal sections (15»m thick) stained with Nissl's stain were then prepared for microscopic examination. Control animals were treated in the same fashion using saline instead of aFGF. Control animals showed severe brain damage with necrosis of the pyramidal cells in the hippocampal CA1 layer (Figs. 9 and 10). Similar lesions were not observed in the brains of animals, given aFGF (Figs. 8 and 11).

### Example 2

Overnight-fasted male rats (Sprague-Dawley strain) weighing 250g were provided with commercial rat chow or treated with glucose intraperitoneally (2.0g/kg body weight). The changes in cerebrospinal fluid concentrations of aFGF were determined by a bioassay using the specific behavioural responses of hydra japonica to aFGF.

The results which are shown in Figure 4, suggest that in the rat, the secretion of aFGF after intraperitoneal glucose administration, was essentially lower and slower than the secretion of aFGF which occurs after eating.

In addition, in rats intraperitoneally administered with glucose with or without L-arginine, the concentrations of aFGF in the cerebrospinal fluid increased 2 hours after treatment. A similar effect was observed in the case of use of a mixture of branched chain L-amino acids, leucine, isoleucine and valine. This data is shown in Figure 5. The data indicates that these nutrients can cause the release of aFGF in the brain linearly with respect to nutrient dosage (i.e. a typical linear dose-response plot).

### Example 3

The anorexigenic effect of aFGF administered intra-third ventricularly to overnight-fasted rats (220ng/rat) was examined. The treatment with aFGF significantly reduced dietary consumption. The results are shown in Figure 2.

Neuronal recordings from glucose-sensitive neurons in the lateral hypothalamic area (feeding centre) of conscious rats show that spontaneous discharge as a measure of neural activity was severely suppressed after a latency of 8.5 minutes by the iontophoretic application of aFGF. In contrast, the application of glutamic acid caused no such effect. These results are shown in Figure 3.

These results show that aFGF has the ability to strongly suppress neuronal activity.

### Example 4

Male mice, weighing 40g, were acclimatised to specially designed cages having separate light and dark compartments. On the second day, animals were pretreated by the intraperitoneal administration of glucose (2g/kg body weight) with or without L-arginine (2.1g/kg body weight). Two hours later, the animals were settled in the light compartments. On movement into the dark compartments, a weak electric shock was administered. On the third day, the mice were again settled in the light compartments and the time taken (i.e. the latency period) to move from the light compartment to the dark compartment was determined as an evaluation of memory retention. Data from this passive avoidance test is shown in Figure 6. The data indicates that pretreatment with glucose significantly enhanced memory retention in mice, and that the administration of glucose in combination with L-arginine was much more effective in this respect than the administration of glucose alone.

### Example 5

The effect of constant administration of glucose with or without L-arginine was also examined as described for aFGF treatment in Example 1. The number of necrotic pyramidal cells in the hippocampal CA1 layer for each test and control group (N=10) were counted microscopically.

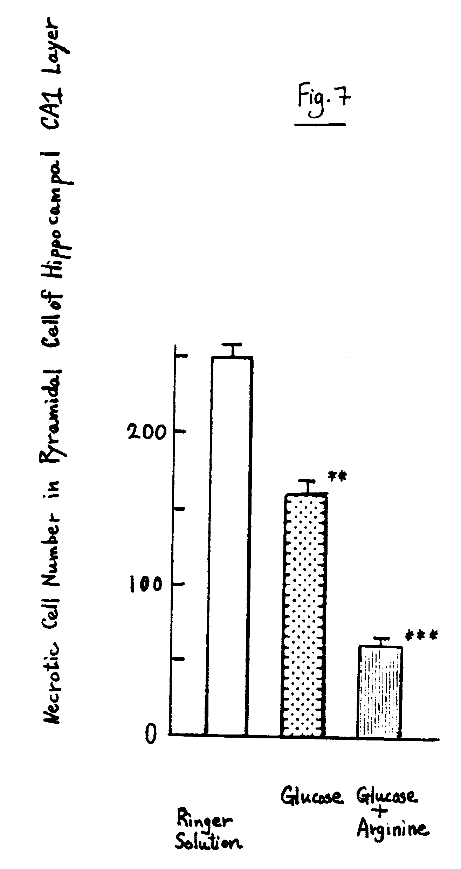

The results are shown in Figure 7. The results indicate that treatment with glucose improved the survival rate of the pyramidal cells after short term brain ischaemia (p<0.01 vs saline control). Concomitant administration with L-arginine was even more effective than treatment with glucose alone (p<0.01 vs glucose alone, p,0.001 vs saline control).

During spasms caused by brain ischaemia and hypoglycaemia, concentrations of glutamic and/or aspartic acids rise. The rise in concentration of these amino acids damages neurons with consequent loss of function. This produces the characteristic symptoms of senile dementia, memory loss and other related conditions. The drugs as provided by the present invention can be used to effectively treat or ameliorate these conditions, as the drugs seem to decrease neuron sensitivity (i.e. increase tolerance) to glutamic and/or aspartic acids. Furthermore the drugs may aid neuronal repair and remodelling of neuronal pathways after ischaemic and hypoglycaemic episodes.

## Claims (Claims for the following Contracting State(s): AT, BE, CH, DE, FR, GB, IT, LI)

1. Use of acidic fibroblast growth factor (aFGF) or a physiologically active fragment or peptide analogue thereof in the preparation of a medicament for treating senile dementia, memory disorders and associated conditions.

2. Use of glucose and/or one or more L-amino acids which cause aFGF secretion in the brain and peripheral tissues, in the preparation of a medicament for treating senile dementia, memory disorders and associated conditions.

3. The use according to claim 2 wherein the L-amino acid is selected from one or more of arginine, leucine, isoleucine and valine.

4. A pharmaceutical composition comprising
(i) aFGF, a physiologically active fragment or peptide analogue thereof or an activator of protein kinase C; and
(ii) glucose and/or one or more L-amino acids which cause aFGF secretion in the brain and peripheral tissues;
arranged for simultaneous or sequential administration.

5. The pharmaceutical composition according to claim 4 wherein said activator of protein kinase C is phorbol ester.

6. The pharmaceutical composition according to claim 4 or claim 5 wherein said L-amino acid of (ii) is selected from one or more of arginine leucine, isoleucine and valine.

7. The pharmaceutical composition according to any one of claims 4, 5 and 6 wherein at least one of (i) and (ii) is adapted for intravenous administration.

8. The pharmaceutical composition according to any one of claims 4, 5 and 6 wherein at least one of (i) and (ii) is adapted for oral administration.

## Claims (Claims for the following Contracting State(s): ES)

1. A method for the production of a medicament for treating senile dementia, memory disorders and associated conditions comprising using of acidic fibroblast growth factor (aFGF) or a physiologically active fragment or peptide analogue thereof.

2. A method for the production of a medicament for treating senile dementia, memory disorders and associated conditions comprising using glucose and/or one or more L-amino acids which cause a FGF secretion in the brain and peripheral tissues.

3. The method according to claim 2 wherein the L-amino acid is selected from one or more of arginine, leucine, isoleucine and valine.

4. A method for the production of a pharmaceutical composition comprising mixing (i) aFGF, a physiologically active fragment or peptide analogue thereof or an activator of protein kinase C; and (ii) glucose and/or one or more L-amino acids which cause aFGF secretion in the brain and peripheral tissues;
arranged for simultaneous or sequential administration.

5. The method according to claim 4 wherein said activator of protein kinase C is a phorbol ester.

6. The method according to claim 4 or claim 5 wherein said L-amino acid of (ii) is selected from one or more of arginine leucine, isoleucine and valine.

7. The method according to any one of claims 4, 5 and 6 wherein at least one of (i) and (ii) is adapted for intravenous administration.

8. The method according to any one of claims 4, 5 and 6 wherein at least one of (i) and (ii) is adapted for oral administration.

## Patentansprüche (Patentansprüche für folgende(n) Vertragsstaat(en): AT, BE, CH, DE, FR, GB, IT, LI)

1. Verwendung eines sauren Fibroblasten-Wachstumsfaktors (aFGF) oder eines physiologisch aktiven Fragments oder Peptid-Analogons davon bei der Herstellung eines Arzneimittels zur Behandlung von Altersdemenz, Gedächtnisstörungen und damit in Zusammenhang stehenden Zuständen.

2. Verwendung von Glucose und/oder einer oder mehr L-Aminosäuren, welche die aFGF-Sekretion im Gehirn und peripheren Geweben verursachen, bei der Herstellung eines Arzneimittels zur Behandlung von Altersdemenz, Gedächtnisstörungen und damit in Zusammenhang stehenden Zuständen.

3. Verwendung nach Anspruch 2, wobei die L-Aminosäure unter einer oder mehr der Aminosäuren Arginin, Leucin, Isoleucin und Valin ausgewählt ist.

4. Arzneimittelzusammensetzung, enthaltend
(i) aFGF, ein physiologisch aktives Fragment oder Peptid-Analogon davon oder einen Aktivator von Proteinkinase C und
(ii) Glucose und/oder eine oder mehr L-Aminosäuren, welche die aFGF-Sekretion im Gehirn und peripheren Geweben verursachen, zubereitet für die gleichzeitige oder aufeinanderfolgende Verabreichung.

5. Arzneimittelzusammensetzung nach Anspruch 4, wobei der Aktivator für Proteinkinase C Phorbolester ist.

6. Arzneimittelzusammensetzung nach Anspruch 4 oder Anspruch 5, wobei die L-Aminosäure in (ii) eine oder mehr, ausgewählt unter Arginin, Leucin, Isoleucin und Valin ist.

7. Arzneimittelzusammensetzung nach einem der Ansprüche 4, 5 und 6, wobei mindestens eines unter (i) und (ii) für die intravenöse Verabreichung zubereitet ist.

8. Arzneimittelzusammensetzung nach einem der Ansprüche 4, 5 und 6, wobei mindestens eines von (i) und (ii) für die orale Verabreichung zubereitet ist.

## Patentansprüche (Patentansprüche für folgende(n) Vertragsstaat(en): ES)

1. Verfahren zur Herstellung eines Arzneimittels zur Behandlung von Altersdemenz, Gedächtnisstörungen und damit in Zusammenhang stehenden Zuständen, welches die Verwendung von saurem Fibroblasten-Wachstumsfaktor (aFGF) oder eines physiologisch aktiven Fragments oder Peptid-Analogons davon umfasst.

2. Verfahren zur Herstellung eines Arzneimittels zur Behandlung von Altersdemenz, Gedächtnisstörungen und damit in Zusammenhang stehenden Zuständen, welches die Verwendung von Glucose und/oder einer oder mehr L-Aminosäuren, welche die aFGF-Sekretion im Gehirn und peripheren Geweben verursachen, umfaßt.

3. Verfahren nach Anspruch 2, wobei die L-Aminosäure eine oder mehr, ausgewählt unter Arginin, Leucin, Isoleucin und Valin ist.

4. Verfahren zur Herstellung einer Arzneimittelzusammensetzung, welches das Vermischen von (i) aFGF, einem physiologisch aktiven Fragment oder Peptid-Analogon davon oder eines Aktivators für Proteinkinase C und (ii) Glucose und/oder einer oder mehr L-Aminosäuren, welche aFGF-Sekretion im Gehirn und peripheren Geweben verursachen, in der Weise umfaßt, daß diese zur gleichzeitigen oder aufeinanderfolgenden Verabreichung geeignet sind.

5. Verfahren nach Anspruch 4, wobei der Aktivator für Proteinkinase C Phorbolester ist.

6. Verfahren nach Anspruch 4 oder Anspruch 5, wobei die L-Aminosäure in (ii) eine oder mehr, ausgewählt unter Arginin, Leucin, Isoleucin und Valin ist.

7. Verfahren nach einem der Ansprüche 4, 5 und 6, wobei mindestens eines von (i) und (ii) für die intravenöse Verabreichung zubereitet ist.

8. Verfahren nach einem der Ansprüche 4, 5 und 6, wobei mindestens eines von (i) und (ii) für die orale Verabreichung zubereitet ist.

## Revendications (Revendications pour l'(les) Etat(s) contractant(s) suivant(s): AT, BE, CH, DE, FR, GB, IT, LI)

1. Utilisation du facteur de croissance des fibroblastes acide (aFGF) ou d'un fragment physiologiquement actif ou d'un analogue peptidique de ce dernier pour la préparation d'un médicament destiné au traitement de la démence sénile, des troubles mnésiques et des tableaux cliniques associés.

2. Utilisation de glucose et/ou d'un ou plusieurs acides aminés de configuration L qui induisent la sécrétion d'aFGF dans le cerveau et les tissus périphériques pour la préparation d'un médicament destiné au traitement de la démence sénile, des troubles mnésiques et des tableaux cliniques associés.

3. Utilisation selon la revendication 2, dans laquelle l'acide aminé de configuration L est un ou plusieurs des suivants : arginine, leucine, isoleucine et valine.

4. Composition pharmaceutique comprenant
(i) de l'aFGF, un fragment physiologiquement actif ou un analogue peptidique de ce dernier ou un activateur de la protéine-kinase C et
(ii) du glucose et/ou un ou plusieurs acides aminés de configuration L qui induisent la sécrétion d'aFGF dans le cerveau et les tissus périphériques,
conçus pour une administration simultanée ou séquentielle.

5. Composition pharmaceutique selon la revendication 4, dans laquelle ledit activateur de la protéine-kinase C est un ester de phorbol.

6. Composition pharmaceutique selon la revendication 4 ou 5, dans laquelle ledit acide aminé à la configuration L de (ii) est un ou plusieurs des suivants : arginine, leucine, isoleucine et valine.

7. Composition pharmaceutique selon l'une quelconque des revendications 4, 5 et 6, dans laquelle au moins une des substances de (i) et (ii) convient à l'administration intraveineuse.

8. Composition pharmaceutique selon l'une quelconque des revendications 4, 5 et 6, dans laquelle au moins une des substances de (i) et (ii) convient à l'administration orale.

## Revendications (Revendications pour l'(les) Etat(s) contractant(s) suivant(s): ES)

1. Procédé de production d'un médicament destiné au traitement de la démence sénile, des troubles mnésiques et des tableaux cliniques associés, comprenant l'utilisation du facteur de croissance des fibroblastes acide (aFGF) ou d'un fragment physiologiquement actif ou d'un analogue peptidique de ce dernier.

2. Procédé de production d'un médicament destiné au traitement de la démence sénile, des troubles mnésiques et des tableaux cliniques associés, comprenant l'utilisation de glucose et/ou d'un ou plusieurs acides aminés de configuration L qui induisent la sécrétion d'aFGF dans le cerveau et les tissus périphériques.

3. Procédé selon la revendication 2, dans lequel l'acide aminé de configuration L est un ou plusieurs des suivants : arginine, leucine, isoleucine et valine.

4. Procédé de production d'une composition pharmaceutique comprenant le mélange (i) d'aFGF, d'un fragment physiologiquement actif ou d'un analogue peptidique de ce dernier ou d'un activateur de la protéine-kinase C et (ii) de glucose et/ou d'un ou plusieurs acides aminés de configuration L qui induisent la sécrétion d'aFGF dans le cerveau et les tissus périphériques,
conçus pour une administration simultanée ou séquentielle.

5. Procédé selon la revendication 4, dans lequel ledit activateur de la protéine-kinase C est un ester de phorbol.

6. Procédé selon la revendication 4 ou 5, dans lequel ledit acide aminé de configuration L de (ii) est un ou plusieurs des suivants : arginine, leucine, isoleucine et valine.

7. Procédé selon l'une quelconque des revendications 4, 5 et 6, dans lequel au moins une des substances de (i) et (ii) convient à l'administration intraveineuse.

8. Procédé selon l'une quelconque des revendications 4, 5 et 6, dans lequel au moins une des substances de (i) et (ii) convient à l'administration orale.
